# EUROPEAN PATENT SPECIFICATION

(11) **EP 1 386 593 B1**
(45) Date of publication and mention of the grant of the patent: **30.03.2005**
(21) Application number: 03021907.5
(22) Date of filing: 17.01.1997
(51) Int. Cl.: A61F 13/00

(54) **Epidermal positioning mechanism**
Nasal epidermaler Einbringmechanismus
Mécanisme de disposition de couches d'épiderme

(30) Priority: 10.05.1996 US 17258 P
(43) Date of publication of application: 04.02.2004
(62) Divisional of application: 97904815.4
(73) Proprietor: Beaudry, Wallace J., Elkhart Lake, WI 53020 (US)
(72) Inventor: Beaudry, Wallace J., Elkhart Lake, WI 53020 (US)
(74) Representative: Sparing, Rolf Klaus

(56) References cited:
- US-A- 3 085 024

## Description

The invention relates to an epidermal positioning mechanism in accordance to the preamble of claim 1.

US 3,085,024 describes an epidermal positioning mechanism for the treatment of wounds, comprising a bandage structure which is fastened by a first elastic material and a second elastic material each designed as a tape and each containing a plurality of perforations forming tear zones.

It is the object of the invention to provide an epidermal positioning mechanism, which permits a safe and persistent holding on an epidermal area.

The invention achieves this object for the epidermal positioning mechanism mentioned at the beginning by the characterizing features of claim 1. Accordingly, the present invention may be considered an epidermal positioning mechanism having an elastic material coupled to a first end piece and a second piece. The first and second end pieces each having at least one side including an adhesive material. Preferably, but not necessarily, depending upon the application of the present invention, at least one of the end pieces would be the anchoring structure or mechanism while the other end piece acts as a lifting end piece.

Additionally the present invention need not solely be used as a nasal dilator but, as previously noted, may also be used as an epidermal positioning system for treatments of wounds and incisions by either keeping the wound or incision open for the purpose of medical treatment such as surgical procedures or cleansing of the wound or incision or by positioning the ends of the wound together in close proximity to aid in suturing of a wound or simply to be used as a suture mechanism in and of itself to hold the ends of a wound together or to hold the ends of an incision together.

Further, when the device of the present invention is used over a wound it may also have application as a bandage. For example, the elastic or resilient material will have at least one side positioned over and adjacent the wound or incision area. This side positioned over or adjacent the wound or incision area may have a medicinal material applied thereto. This medicinal material may be, for example, zinc chromate or an alginate like calcium or sodium alginate; each of those materials respectively having anti-bacterial and clot enhancing capabilities. Other medicinal materials or even non-medicinal materials could also be applied using the device of the present invention depending upon the goals and results desired of the particular user.

If the epidermal positioning mechanism of the present invention is used as a bandage it should be noted that a bandage structure could be combined with the present invention such that the bandage structure would have at least a first end and second end and elastic material would be coupled to the first end and to the second end with an anchoring structure coupled to a portion of the elastic material as well. This would provide at least two anchor points at the ends of the resilient elastic material not coupled to the bandage structure. In this manner one of the anchor structures could be adhered to the skin at a predetermined position and the bandage structure positioned over the wound or incision by stretching the resilient or elastic material and then applying the other anchor structure could be to the skin at another predetermined position. In this manner, the elastic material will contract and this will have the effect of forcing the bandage material into more positive contact with the wound and thereby enhance the effectiveness of the bandaged material. If desired a medicinal compound could be applied to the surface of the bandage material which is adjacent to the surface of the wound or incision.

The anchoring structure in such a use would of course comprise at least two end pieces coupled to the elastic material at predetermined positions and the end pieces would include an adhesive material attached to a side of the anchoring end pieces adjacent to the epidermis or skin to which they are to be attached. The bandage structure could also have a medicinal material applied to it as previously noted with respect to the elastic material.
Figure 1 is a perspective view of an another alternative structure of the present invention.
Figure 2 is a top plan view of the an alternative embodiment to the structure disclosed in figure 1.

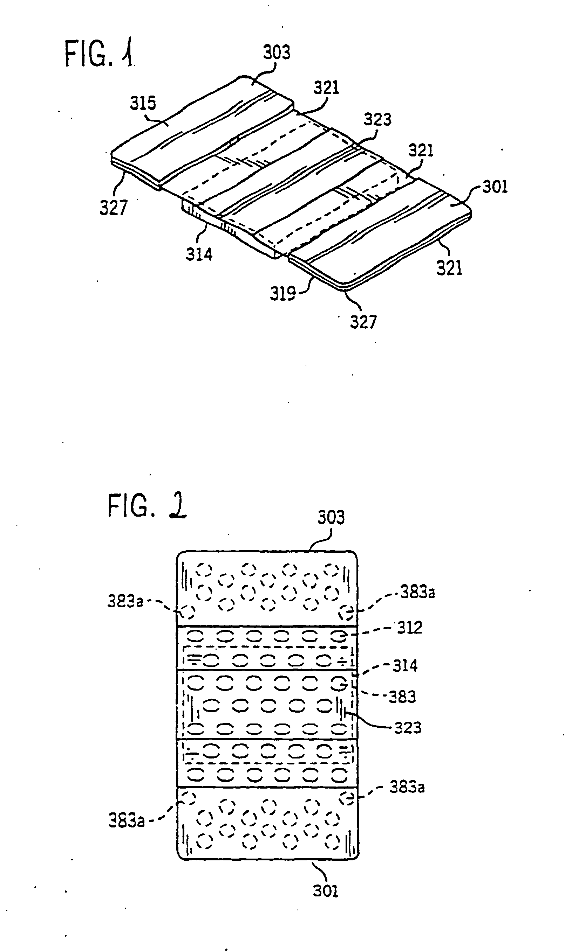

Referring to Figure 1 a very simple version of the present invention is illustrated. In this embodiment the dressing 300 is composed of a piece of latex 321 having two ends to which anchors 301 and 303 are respectively attached using an adhesive. The ends of the latex 321 are simply sandwiched between the layers 315 and 319. A piece of stiffening material 323 is glued across the mid-section of the latex 321 and pad 314 is glued to the underside of the latex 321 as illustrated. The bottom side of each respective anchor section 301 and 303 having an adhesive 327 applied thereto.

Referring to Figure 2 illustrates the embodiment of Figure 53 with the addition of a series of openings 383 being applied to the entire dressing 300. Depending upon the material through which the opening 383 is made the function of the opening will vary. Openings 312 in the latex 321 will act to vary the elasticity of the latex. Openings 383a will create stress points and help maintain the dressing 300 in a straight alignment between its anchors 301 and 303. Openings 383b will allow air access to the treatment area.

The foregoing is considered as illustrative only of the principles of the invention. Furthermore, since numerous modifications and changes will readily occur to those skilled in the art, it is not desired to the invention to the exact construction and operation shown and described. While the preferred embodiment has been described, the details may be changed without departing from the invention, which is defined by the claims.

## Claims

1. An epidermal positioning mechanism, comprising
a bandage structure (314) having at least a first end and a second end,
a first elastic material (321) coupled to the first end of the bandage structure (314) and
a second elastic material (321) coupled to the second end of the bandage structure (314) ,
**characterized in**
**that** a first anchoring structure (301) is coupled to at least a portion of the first elastic material (321 ), and
**that** a second anchoring structure (303) is coupled to at least a portion of the second elastic material (321 ).

2. The epidermal positioning mechanism according to claim 1 wherein the first anchoring structure (301) and the second anchoring structure (303) each comprises an end piece coupled to the respective fist and second elastic material (321), the end pieces including an adhesive material (327).

3. The epidermal positioning mechanism according to claim 1 or 2 wherein the bandage structure (323) includes at least one side having a medicinal material thereon.

4. The epidermal positioning mechanism according to claim 3 wherein the medicinal material comprises zinc chromate.

5. The epidermal positioning mechanism according to claim 3 wherein the medicinal material comprises zinc chromate impregnated in a hydrocolloid material.

6. The epidermal positioning mechanism according to claim 3 wherein the medicinal material comprises an alginate.

7. The epidermal positioning mechanism according to claim 6 wherein the alginate comprises of one of the group consisting of calcium alignate and sodium alginate.

## Patentansprüche

1. Epidermischer Hebemechanismus, umfassend
eine Verbandstruktur (314), die wenigstens ein erstes Ende und ein zweites Ende aufweist,
ein erstes elastisches Material (321), das mit dem ersten Ende der Verbandstruktur (314) gekoppelt ist, und ein zweites elastisches Material (321), das mit dem zweiten Ende der Verbandstruktur (314) gekoppelt ist,
**dadurch gekennzeichnet,**
**dass** eine erste Verankerungsstruktur (301) mit wenigstens einem Teilbereich des ersten elastischen Materials (321) gekoppelt ist, und
**dass** eine zweiter Verankerungsstruktur (303) mit wenigstens einem Teilbereich des zweiten elastischen Materials (321) gekoppelt ist.

2. Epidermische Hebevorrichtung nach Anspruch 1, wobei die erste Verankerungsstruktur (301) und die zweite Verankerungsstruktur (303) jeweils ein Endstück aufweisen, das jeweils mit dem ersten bzw. dem zweiten elastischen Material (321) gekoppelt ist, wobei die Endstücke ein selbstklebendes Material (327) aufweisen.

3. Epidermische Hebevorrichtung nach Anspruch 1 oder 2, wobei die Verbandstruktur (323) wenigstens eine Seite aufweist, auf der ein medizinisches Material vorgesehen ist.

4. Epidermische Hebevorrichtung nach Anspruch 3, wobei das medizinische Material Zink-Chromat umfasst.

5. Epidermische Hebevorrichtung nach Anspruch 3, wobei das medizinische Material Zink-Chromat umfasst, das auf einem hydrocolloiden Material imprägniert ist.

6. Epidermische Hebevorrichtung nach Anspruch 3, wobei das medizinische Material ein Alginat umfasst.

7. Epidermische Hebevorrichtung nach Anspruch 6, wobei das Alginat eins von Calcium-Alginat und Sodium-Alignat umfasst.

## Revendications

1. Un mécanisme de disposition de couches d'épiderme, comprenant
une structure de bandage (314) ayant au moins une première extrémité et une deuxième extrémité,
un premier matériau élastique (321) couplé à la première extrémité de la structure de bandage (314) et
un deuxième matériau élastique (321) couplé à la deuxième extrémité de la structure de bandage (314),
**caractérisé en ce que**
une première structure d'ancrage (301) est couplée à au moins une partie du premier matériau élastique (321), et
**en ce que** une deuxième structure d'ancrage (303) est couplée à au moins une partie du deuxième matériau élastique (321).

2. Le mécanisme de disposition de couches d'épiderme selon la revendication 1, dans lequel la première structure d'ancrage (301) et la deuxième structure d'ancrage (303) comprennent chacune une pièce d'extrémité couplée respectivement au premier et deuxième matériau élastique (321), les pièces d'extrémité comprenant un matériau adhésif (327).

3. Le mécanisme de disposition de couches d'épiderme selon l'une des revendications 1 ou 2, dans lequel la structure de bandage (323) comprend au moins un côté ayant un matériau médicinal.

4. Le mécanisme de disposition de couches d'épiderme selon la revendication 3, dans lequel le matériau médicinal comprend du chromate de zinc.

5. Le mécanisme de disposition de couches d'épiderme selon la revendication 3, dans lequel le matériau médicinal comprend du chromate de zinc imprégné dans un matériau hydrocolloïde.

6. Le mécanisme de disposition de couches d'épiderme selon la revendication 3, dans lequel le matériau médicinal comprend un alginate.

7. Le mécanisme de disposition de couches d'épiderme selon la revendication 6, dans lequel l'alginate comprend un produit dans le groupe consistant en l'alginate de calcium et l'alginate de sodium.
